(19) Europäisches Patentamt
European Patent Office
Office européen des brevets

(11) Veröffentlichungsnummer: **0 023 686**
**B1**

# (12) EUROPÄISCHE PATENTSCHRIFT

(45) Veröffentlichungstag der Patentschrift: **31.10.84**

(21) Anmeldenummer: **80104405.8**

(22) Anmeldetag: **26.07.80**

(51) Int. Cl.³: **C 07 C 69/54,** C 07 C 67/08, C 07 C 41/03, A 61 K 6/08, C 09 J 3/14, C 08 F 120/28 // C07C43/11, C07C43/13

## (54) (Meth)acrylsäureester von Ethergruppen enthaltenden tricyclischen Decandiolen, Verfahren zu ihrer Herstellung und Verwendung zur Herstellung von Kleb- oder Dichtungsmitteln.

(30) Priorität: **07.08.79 DE 2931925**

(43) Veröffentlichungstag der Anmeldung: **11.02.81 Patentblatt 81/6**

(45) Bekanntmachung des Hinweises auf die Patenterteilung: **31.10.84 Patentblatt 84/44**

(84) Benannte Vertragsstaaten: **AT BE CH DE FR GB IT LI NL SE**

(56) Entgegenhaltungen:
**EP - A - 0 023 685**
**DE - A - 2 301 067**
**DE - A - 2 816 823**
**US - A - 4 115 346**

**CHEMICAL ABSTRACTS, Band 78, No. 20, 21-05-1973, Seite 36, Zusammenfassung 125364p**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Schmitz-Josten, Robert, Dr., Gerstenkamp 6, D-5000 Koeln 80 (DE)**
Erfinder: **Dietrich, Manfred, Dr., c./o. Mobay Chemical Corporation, New Martinsville 26155 West Virginia (US)**
Erfinder: **Bömer, Bruno, Dr., Gustav-Freytag-Strasse 2, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Gegenstand der Erfindung sind neue Acrylsäureester und Methacrylsäureester von Ethergruppen enthaltenden tricyclischen Decandiolen, deren Herstellung und deren Verwendung.

Aus der DE-A 2 200 021 sind die Diacrylsäureester und die Dimethacrylsäureester der Di-(hydroxymethyl)-tricyclo-[5.2.1.$0^{2.6}$]-decane bekannt. Die Verwendung dieser Ester für die Herstellung von Dentalmassen ist in der DE-A 2 816 823 beschrieben. Dentalformkörper, welche aus diesen Monomeren hergestellt wurden, weisen jedoch nicht voll befriedigende mechanische Eigenschaften (z. B. Biegefestigkeit und Zugfestigkeit) auf. Unbefriedigend ist auch der bei der Polymerisation auftretende Schrumpf. In US-A 4 115 346 werden unter anderem Umsetzungsprodukte von Bisphenol-A-diglycidylether mit Hydroxyalkylacrylaten oder Hydroxyalkylmethacrylaten und deren Verwendung als polymerisierbare Monomere für Dentalmassen beschrieben. Monomere dieser Art weisen jedoch – wie in der Literaturstelle selbst beschrieben wird und wie es auch in der DE-A 2 816 823 diskutiert wird – eine unerwünscht hohe Quellbarkeit in Wasser auf, welche für dentale Anwendungszwecke von Nachteil ist. Ein weiterer Nachteil liegt in der extrem hohen Viskosität der Monomeren, welche um eine Größenordnung höher ist als bei den Verbindungen gemäß DE-A 2 200 021.

In Monomere mit derart hoher Viskosität kann keine für dentale Anwendungszwecke ausreichend hohe Menge an anorganischen Füllstoffen eingearbeitet werden, ohne daß die für die Verarbeitung erforderliche pastenartige Konsistenz verloren geht. In US-A 4 115 346 wird daher vorgeschlagen (Beispiel 9), als Verdünnungsmittel eine größere Menge an Methylmethacrylat mitzuverwenden. Durch diese Maßnahme geht jedoch der Vorteil des geringen Polymerisationsschrumpfes des hochviskosen Monomeren wieder verloren; darüber hinaus ist auch der hohe Dampfdruck des verwendeten Verdünnungsmittels bei Zahnfüllmaterialien unerwünscht.

Es wurde nun gefunden, daß Acryl- bzw. Methacrylsäureester von Ethergruppen enthaltenden tricyclischen Decandiolen der allgemeinen Formel

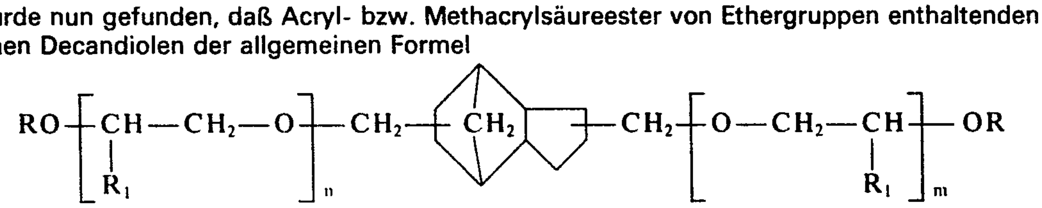

in welcher

R für $CH_2{=}CH{-}CO{-}$ oder $CH_2{=}\underset{CH_3}{\underset{|}{C}}{-}CO{-}$

$R_1$ für H, $CH_3$ oder $C_2H_5$ und
n + m für 1–10

stehen, in hervorragender Weise für die Herstellung von Dentalmassen geeignet sind.

Weiterhin wurde gefunden, daß man die erfindungsgemäßen Acryl- bzw. Methacrylsäureester erhält, indem man 3,8-,3,9- oder 4,8-Di-(hydroxymethyl)-tricyclo-[5.2.1.$0^{2.6}$]-decane oder deren Gemische zunächst mit 1–10 Mol eines Alkylenoxids der allgemeinen Formel

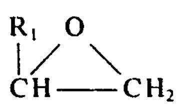 (II)

in der

$R_1$ für H, $CH_3$ oder $C_2H_5$

steht, umsetzt und die dabei entstehenden Verbindungen der allgemeinen Formel (I)

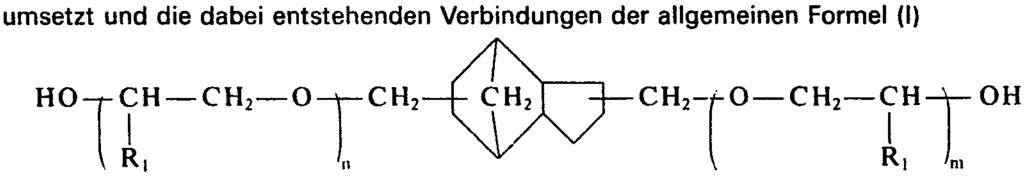 (I)

in an sich bekannter Weise in ihre Acryl- bzw. Methacrylsäureester überführt.

Als Ausgangsmaterial zur Herstellung der erfindungsgemäßen Verbindungen dienen die in 3,8- bzw. 3,9- oder 4,8-Stellung Hydroxymethylgruppen enthaltenden Tricyclo-[5.2.1.$0^{2.6}$]-decane der allgemeinen Formel

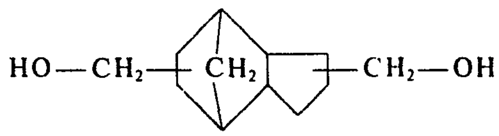

die in an sich bekannter Weise durch Oxosynthese aus Kohlenmonoxid und Dicyclopentadien in Gegenwart von Kobalt-Katalysatoren und anschließendes Hydrieren der gebildeten Tricyclodecan-dialdehyde erhältlich sind, wobei ein Gemisch der Isomeren entsteht. Dieses Gemisch wird als TCD-DM bezeichnet.

Diese Ausgangsmaterialien werden mit n + m = 1–10 Mol eines niederen Alkylenoxids, wie Ethylenoxid, Propylenoxid oder Butylenoxid bei Temperaturen von 70–150°C, vorzugsweise bei 100–120°C, in Gegenwart von basischen Katalysatoren zu Verbindungen der Formel I umgesetzt, wobei das Alkylenoxid zweckmäßigerweise nach Maßgabe seines Verbrauchs zudosiert wird.

Als basische Katalysatoren werden vorzugsweise Alkali- oder Erdalkali-Alkoholate verwendet, welche auch in situ hergestellt werden können.

Zur Herstellung der erfindungsgemäßen Acryl- bzw. Methacrylsäureester aus den tricyclischen Diolen der Formel I können prinzipiell alle zur Esterbildung brauchbaren Reaktionen herangezogen werden. Im einfachsten Falle wird das tricyclische Diol mit Acryl- bzw. Methacrylsäure, insbesondere unter Zugabe von Katalysatoren verestert, wobei man das gebildete Wasser unter Verwendung von Lösungsmitteln azeotropisch aus dem Reaktionsgemisch entfernen kann. Anstelle der Säuren können natürlich auch deren Anhydride oder Säurehalogenide eingesetzt werden. Auch eine Umesterung mit beispielsweise den Methylestern oder Ethylestern der Acrylsäure oder Methacrylsäure ist möglich. Hierbei werden Umesterungskatalysatoren, wie Ester der Titansäure oder Dibutylzinnoxid verwendet. Prinzipiell kann man natürlich die tricyclischen Diole auch mit einer leicht flüchtigen Säure, z. B. Essigsäure, verestern und anschließend eine Umesterung mit dem Acryl- bzw. Methacrylsäureester eines leicht flüchtigen Alkohols, wie z. B. Acrylsäuremethylester oder Methacrylsäuremethylester durchführen.

Bei der Herstellung der erfindungsgemäßen Acryl- bzw. Methacrylsäureester sollten Polymerisationsinhibitoren zur Vermeidung einer vorzeitigen Polymerisation anwesend sein. Geeignete Inhibitoren sind als Stabilisatoren, für Monomere bekannte Substanzen, wie etwa Hydrochinon, dialkylierte Phenole, Chinone, aromatische Nitroverbindungen, Phenothiazin oder Methylenblau. In manchen Fällen ist es ausreichend, wenn man für eine reichliche Zufuhr von als Polymerisationsinhibitor wirkendem Sauerstoff Sorge trägt.

Die erfindungsgemäßen Acryl- bzw. Methacrylsäureester sind Monomere, die mittels bekannter Initiatoren in hochmolekulare, unlösliche Verbindungen überführt werden können. Sie sind z. B. infolge ihrer relativ niedrigen Viskosität hervorragend geeignet zur Herstellung hochgefüllter Zahnreparaturmaterialien. Ferner eignen sie sich zur Herstellung von bei Sauerstoffausschluß erhärtenden Gemischen, die als Kleb- oder Dichtungsmittel Verwendung finden.

## Beispiele

### Vorschrift zur Herstellung der oxyalkylierten Bis-hydroxy-methyl-tricyclo-[5.2.0.1.$^{2.6}$]-decane (TCD-DM)

### Produkt A: TCD-DM + 2 Mol Ethylenoxid

In einem heizbaren Rührautoklaven, der mit einer Vorrichtung zur Azeotrop-Entwässerung versehen ist, werden 4830 g Bishydroxymethyl-tricyclo-[5.2.0.1.$^{2.6}$]-decan (TCD-DM; Gemische der 3,8-, 3,9- und 4,8-Isomeren) und 600 g Toluol vorgelegt und die Luft gegen Stickstoff ausgetauscht. Man gibt bei 80°C 70 g 50%ige wäßrige Kaliumhydroxidlösung zu und entfernt bei 100–115°C 46 g Wasser aus dem Reaktionsgemisch durch azeotrope Destillation. Anschließend werden bei 100–115°C und 0,4–0,6 bar 2170 g Ethylenoxid langsam zudosiert und das Gemisch 3 Stunden bei 100–105°C nachgerührt. Das alkalische Reaktionsprodukt wird mit 700 g Wasser und 245 g einer 12,5%igen wäßrigen Schwefelsäure neutralisiert. Anschließend wird nach Zugabe eines Filterhilfsmittels und eines Antioxydans, wie 0,05% 2,6-Bis-t-butyl-p-Kresol das Wasser im Vakuum bei 70–90°C abdestilliert und die abgeschiedenen Salze zusammen mit dem Filterhilfsmittel abfiltriert. Das so erhaltene neutrale Produkt hat eine Hydroxylzahl von 383 und eine Viskosität von $\eta_{25°C}$ = 3550 mPas.

Analog werden folgende Hydroxyalkylierungsprodukte des Bishydroxymethyl-tricyclo-[5.2.0.1.$^{2.6}$]-decans (TCD-DM) hergestellt:

Produkt B: TCD-DM + 4 Mol Ethylenoxid;
Einsatz 3688 g TCD-DM + 3312 g Ethylenoxid; OH-Zahl 289; $\eta_{25°C}$ = 980 mPas.

Produkt C: TCD-DM + 8 Mol Ethylenoxid;
Einsatz 2504 g TCD-DM + 4496 g Ethylenoxid; OH-Zahl 187; $\eta_{25°C}$ = 480 mPas.

Produkt D: TCD-DM + 2 Mol Propylenoxid;
Einsatz 4398 g TCD-DM + 2602 g Propylenoxid; OH-Zahl 341; $\eta_{25°C}$ = 5080 mPas.

Produkt E: TCD-DM + 4 Mol Propylenoxid;
Einsatz 3206 g TCD-DM + 3794 g Propylenoxid; OH-Zahl 255; Viskosität $\eta_{25°C}$ = 1330 mPas.

### Beispiel 1

1460 g Produkt A, 15 g Methylenblau, 50 g p-Toluolsulfosäure, 1150 g destillierte Methacrylsäure und 2000 ml Toluol werden unter Einblasen von Luft zum Sieden erhitzt. Das sich bildende Wasser wird durch azeotrope Destillation ausgekreist. Nach 8 Stunden ist die Wasserabspaltung beendet. Man rührt nach dem Abkühlen 1 Stunde mit 40 g Bleicherde, um das Methylenblau zu adsorbieren und saugt die blaugefärbte Bleicherde ab. Das Filtrat wird mit überschüssiger Natriumcarbonatlösung bis zum pH-Wert von 8 neutralisiert und über ein Filterhilfsmittel wie Zellstoffmehl filtriert. Die obere Phase wird abgetrennt, mit Natriumchloridlösung gewaschen und nach Zusatz eines Polymerisationsinhibitors im Vakuum eingeengt, bis kein Toluol mehr vorhanden ist.

Ausbeute 1817 g; Verseifungszahl 268 (Th. 262); OH-Zahl 5,6; Viskosität $\eta_{25°C}$ = 104 mPas; Brechzahl $n_D^{20}$ = 1,4925.

### Beispiel 2

Methacrylsäureester vom Produkt B

1940 g Produkt B, 15 g Methylenblau, 50 g p-Toluolsulfosäure, 1150 g Methacrylsäure und 2000 ml Toluol werden analog zu Beispiel 1 verestert und aufgearbeitet.

Ausbeute 2364 g; Verseifungszahl 224 (Th. 216); OH-Zahl 2,3; Viskosität $\eta_{25°C}$ = 118 mPas bei 13 dyn/cm$^2$; Brechzahl $n_D^{20}$ = 1,4880.

### Beispiel 3

Methacrylsäureester von Produkt C

Analog zu Beispiel 1 werden aus 3000 g Produkt C erhalten: 2950 g Methacrylsäureester mit folgenden Kennzahlen: Verseifungszahl 154 (Th. 153); OH-Zahl 5,1; Viskosität $\eta_{25°C}$ = 157 mPas; $n_D^{21}$ = 1,4825.

### Beispiel 4

Methacrylsäureester von Produkt D

Aus 3280 g Produkt D werden 4051 g des Methacrylesters erhalten; Verseifungszahl 254; Viskosität $\eta_{25°C}$ = 116 mPas; $n_D^{20}$ = 1,4840.

### Beispiel 5

Methacrylsäureester von Produkt E

Aus 2600 g Produkt E erhält man 2918 g des entsprechenden Methacrylsäureesters; Verseifungszahl 197 (Th. 196); OH-Zahl 9,4; Viskosität $\eta_{25°C}$ = 143 mPas; Brechzahl $n_D^{21}$ = 1,4800.

Analog den Beispielen 1–5 können auch die entsprechenden Acrylsäureester hergestellt werden.

## Patentansprüche

1. Verbindungen der allgemeinen Formel

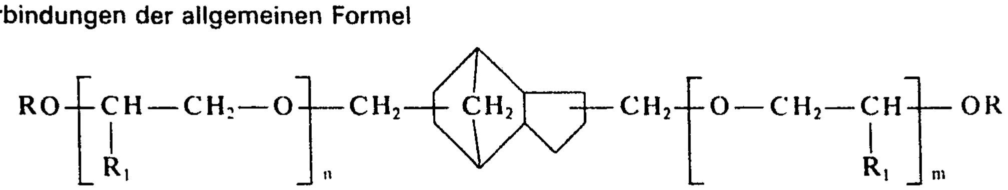

in welcher

R für $CH_2{=}CH{-}CO{-}$ oder $CH_2{=}\underset{CH_3}{\underset{|}{C}}{-}CO{-}$

$R_1$ für H, $CH_3$ oder $C_2H_5$ und
n + m für 1—10

stehen.

2. Verbindungen gemäß Anspruch 1, dadurch gekennzeichnet, daß die Reste $R_1$ beide für Wasserstoff stehen.

3. Verbindungen gemäß Anspruch 1, dadurch gekennzeichnet, daß die Reste $R_1$ beide für eine Methylgruppe stehen.

4. Verbindungen gemäß Anspruch 1 bis 3, dadurch gekennzeichnet, daß sie 2 oder 4 Ethylenoxid- oder Propylenoxid-Einheiten (Summe aus n + m = 2 oder 4) enthalten.

5. Verfahren zur Herstellung von Acryl- bzw. Methacrylsäureestern gemäß Anspruch 1 bis 4, dadurch gekennzeichnet, daß man 3,8-, 3,9- oder 4,8-Di-(hydroxymethyl)-tricyclo-[5.2.1.$0^{2.6}$]-decane oder deren Gemische zunächst mit 1—10 Mol eines Alkylenoxids der allgemeinen Formel

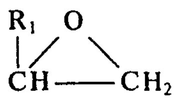

in der

$R_1$ für H, $CH_3$ oder $C_2H_5$

steht, umsetzt und die dabei entstehenden Verbindungen in die Acryl- bzw. Methacrylsäureester überführt.

6. Verfahren nach Anspruch 5, dadurch gekennzeichnet, daß man 3,8-, 3,9- oder 4,8-Di-(hydroxymethyl)-tricyclo-[5.2.1.$0^{2.6}$]-decane oder deren Gemische zunächst mit 1 bis 10 Mol Ethylenoxid umsetzt und die dabei entstehenden Verbindungen in die Acryl- bzw. Methacrylsäureester überführt.

7. Verfahren nach Anspruch 5, dadurch gekennzeichnet, daß man 3,8-, 3,9- oder 4,8-Di-(hydroxymethyl)-tricyclo-[5.2.1.$0^{2.6}$]-decane oder deren Gemische zunächst mit 1 bis 10 Mol Propylenoxid umsetzt und die dabei entstehenden Verbindungen in die Acryl- bzw. Methacrylsäureester überführt.

8. Verwendung der Acryl- bzw. Methacrylsäureester nach Ansprüchen 1 bis 4 zur Herstellung von Zahnreparaturmaterialien.

9. Verwendung der Acryl- bzw. Methacrylsäureester nach Ansprüchen 1 bis 4 zur Herstellung von bei Sauerstoffausschluß erhärtenden Gemischen.

10. Verwendung von Acryl- bzw. Methacrylsäureestern nach Ansprüchen 1 bis 4 zur Herstellung von Kleb- oder Dichtungsmitteln.

## Claims

1. Compounds of the general formula

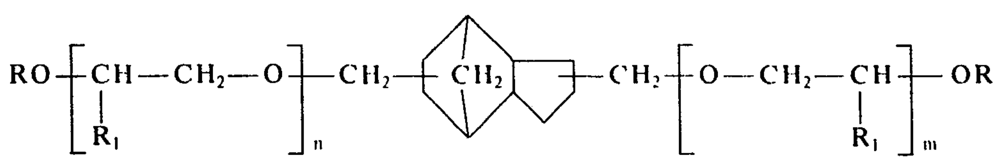

in which

R represents $CH_2{=}CH{-}CO{-}$ or $CH_2{=}\underset{CH_3}{\underset{|}{C}}{-}CO{-}$

$R_1$ represents H, $CH_3$ or $C_2H_5$ and
n + m represent 1—10.

2. Compounds according to Claim 1, characterised in that both radicals $R_1$ represent hydrogen.

3. Compounds according to Claim 1, characterised in that both radicals $R_1$ represent a methyl group.

4. Compounds according to Claim 1 to 3, characterised in that they contain 2 or 4 ethylene oxide or propylene oxide units (sum of $n + m = 2$ or 4).

5. Process for the preparation of acrylic acid esters or methacrylic acid esters according to Claim 1 to 4, characterised in that 3,8-, 3,9- or 4,8-di-(hydroxymethyl)-tricyclo-[5.2.1.$0^{2.6}$]-decanes or mixtures thereof are first reacted with 1—10 moles of an alkylene oxide of the general formula

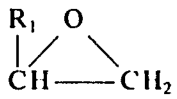

in which

$R_1$ represents H, $CH_3$ or $C_2H_5$,

and the compounds formed thereby are converted into the acrylic acid esters or methacrylic acid esters.

6. Process according to Claim 5, characterised in that 3,8-, 3,9- or 4,8-di-(hydroxymethyl)-tricyclo-[5.2.1.$0^{2.6}$]-decanes or mixtures thereof are first reacted with 1 to 10 moles of ethylene oxide and the compounds formed thereby are converted into the acrylic acid esters or methacrylic acid esters.

7. Process according to Claim 5, characterised in that 3,8-, 3,9- or 4,8-di-(hydroxymethyl)-tricyclo-[5.2.1.$0^{2.6}$]-decanes or a mixture thereof are first reacted with 1 to 10 moles of propylene oxide and the compounds formed thereby are converted into the acrylic acid esters or methacrylic acid esters.

8. Use of the acrylic acid esters or methacrylic acid esters according to Claims 1 to 4 for the preparation of dental repair materials.

9. Use of the acrylic acid esters or methacrylic acid esters according to Claims 1 to 4 for the preparation of mixtures which harden in the absence of oxygen.

10. Use of acrylic acid esters or methacrylic acid esters according to Claims 1 to 4 for the preparation of adhesives or sealing agents.

## Revendications

1. Composés de formule générale

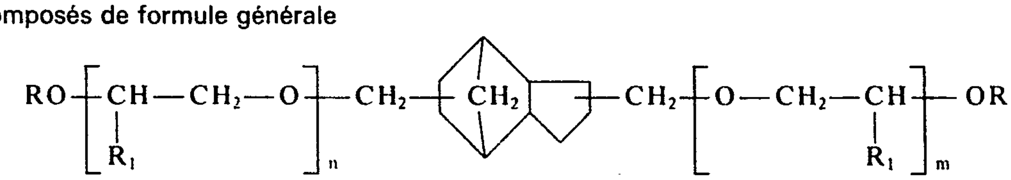

dans laquelle

R représente $CH_2=CH—CO—$ ou 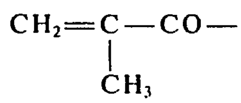

$R_1$ représente H, $CH_3$ ou $C_2H_5$ et
$n + m$ est 1 à 10.

2. Composés selon la revendication 1, caractérisés en ce que les radicaux $R_1$ sont tous deux de l'hydrogène.

3. Composés selon la revendication 1, caractérisés en ce que les radicaux $R_1$ sont tous deux un groupe méthyle.

4. Composés selon les revendications 1 à 3, caractérisés en ce qu'ils contiennent 2 ou 4 unités oxyde d'éthylène ou oxyde de propylène (somme de $n + m = 2$ ou 4).

5. Procédé de fabrication d'esters acryliques ou méthacryliques selon les revendications 1 à 4, caractérisé en ce qu'on fait réagir des 3,8-, 3,9- ou 4,8-di-(hydroxyméthyl)-tricyclo-[5.2.1.$0^{2.6}$]-décanes ou leurs mélanges tout d'abord avec 1 à 10 moles d'un oxyde d'alcoylène de formule générale:

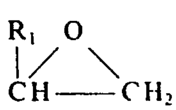

dans laquelle

$R_1$ représente H, $CH_3$ ou $C_2H_5$

et en ce qu'on convertit les composés ainsi obtenus en les esters acryliques ou méthacryliques.

6. Procédé selon la revendication 5, caractérisé en ce qu'on fait réagir des 3,8-, 3,9- ou 4,8-di-(hydroxyméthyl)-tricyclo-[5.2.1.$0^{2,6}$]-décanes ou leurs mélanges d'abord avec 1 à 10 moles d'oxyde d'éthylène et l'on convertit les composés ainsi obtenus en les esters acryliques ou méthacryliques.

7. Procédé selon la revendication 5, caractérisé en ce qu'on fait réagir des 3,8-, 3,9- ou 4,8-di-(hydroxyméthyl)-tricyclo-[5.2.1.$0^{2,6}$]-décanes ou leur mélange d'abord avec 1 à 10 moles d'oxyde de propylène et l'on convertit les composés ainsi obtenus en les esters acryliques ou méthacryliques.

8. Utilisation des esters acryliques ou méthacryliques selon les revendications 1 à 4 pour la fabrication de matériaux de réparation dentaires.

9. Utilisation des esters acryliques ou méthacryliques selon les revendications 1 à 4 pour la fabrication de mélanges durcissant à l'abri de l'oxygène.

10. Utilisation des esters acryliques ou méthacryliques selon les revendications 1 à 4 pour la fabrication d'agents adhésifs ou d'étanchéisation.